# EUROPEAN PATENT APPLICATION

(11) **EP 0 866 132 A2**
(43) Date of publication of application: **23.09.1998**
(21) Application number: 98104907.5
(22) Date of filing: 18.03.1998
(51) Int. Cl.: C12N 15/70, C12N 1/20

(54) **Escherichia coli protease mutant as host cell**

(30) Priority: 19.03.1997 JP 85914/97
(71) Applicant: HSP Research Institute, Inc., Chuo-ku, Osaka-shi, Osaka (JP)
(72) Inventor: Kanemori, Masaaki, Shimogyo-ku, Kyoto-shi, Kyoto (JP); Yanagi, Hideki, Takarazuka-shi, Hyogo-ken (JP); Yura, Takashi, Kyoto-shi, Kyoto (JP)
(74) Representative: Schüssler, Andrea, Dr.

(57) **Abstract**

The *Escherichia coli* mutant carrying a triple deletion mutation in the hslV/U gene, the clpPX gene, and lon gene, and possessing a function to stabilize an unstable protein expressed in *Escherichia coli*, a method for preparing the *Escherichia coli* mutant, a method for stably expressing an unstable protein in *Escherichia coli* by using the *Escherichia coli* mutant, a method for stabilizing an unstable protein derived from *Escherichia coli* by using the *Escherichia coli* mutant, a transformant obtainable by a method comprising introducing an expression vector carrying a gene encoding a foreign protein into the *Escherichia coli* mutant, and a method for preparing a foreign protein using the transformant.

## Description

### BACKGROUND OF THE INVENTION

### Field of the Invention

The present invention relates to an *Escherichia coli* mutant. More specifically, the present invention relates to an *Escherichia coli* mutant possessing a function to stabilize an unstable protein expressed in *Escherichia coli,* a method for preparing the *Escherichia coli* mutant, a method for stably expressing an unstable protein in *Escherichia coli* by using the *Escherichia coli* mutant, a method for stabilizing an unstable protein derived from *Escherichia coli* by using the *Escherichia coli* mutant, a transformant obtainable by a method comprising introducing an expression vector carrying a gene encoding a foreign protein into the *Escherichia coli* mutant, and a method for preparing a foreign protein using the transformant.

### Discussion of the Related Art

The production of useful proteins by expression of foreign genes using *Escherichia coli* can be considered as a basically well-established technique; however, many proteins are rapidly degraded in *Escherichia coli* cells, thereby not always making the expression level of the desired protein satisfactory. As a means for stably expressing an unstable foreign protein in *Escherichia coli*, there has been proposed to use a mutant carrying a mutation in protease genes as a host. In fact, a double mutant carrying mutations in the clpPX gene and the lon gene has been prepared as a host for production of a foreign protein (Japanese Patent Laid-Open No. Hei 8-140671). However, no mutants have yet been obtained so far that are effective in stably expressing various foreign proteins.

An object of the present invention is to provide an *Escherichia coli* mutant possessing a function to stabilize an unstable protein expressed in *Escherichia coli*.

In one embodiment, the present invention provides a method for preparing the *Escherichia coli* mutant.

In another embodiment, the present invention provides a method for stably expressing an unstable protein by using the *Escherichia coli* mutant.

In still another embodiment, the present invention provides a method for stabilizing an unstable protein derived from *Escherichia coli* by using the *Escherichia coli* mutant.

In still another embodiment, the present invention provides a transformant obtainable by a method comprising introducing an expression vector carrying a gene encoding a foreign protein into the *Escherichia coli* mutant.

In still another embodiment, the present invention provides a method for preparing a foreign protein using the transformant.

These and other objects of the present invention will be apparent from the following description.

### SUMMARY OF THE INVENTION

The proteases described above have been known to possess weak but appreciable substrate specificity: Some proteins are degraded by Lon, but not by Clp. It is, therefore, expected to find a novel protease and combine mutations thereof with those of known proteases, to thereby develop a host with a suppressed proteolytic activity that can be useful for the expression of various foreign proteins.

In view of the above, the present inventors have made an attempt to find a novel protease gene, on the basis of the function of degrading a protein showing abnormal folding in *Escherichia coli*, to isolate the hslV/U operon. The nucleotide sequence of the hslV/U operon has already been reported, and the two proteins, HslV and HslU, encoded by the hslV/U operon have been reported to be a catalytic subunit and a regulatory subunit of an ATP-dependent protease, respectively [*Proc. Natl. Acad. Sci. USA* **93**, 5808-5813 (1996); *J. Biol. Chem*. **271**, 14035-14040 (1996)].

The present inventors have found that human prourokinase used as a foreign protein becomes considerably unstable in *Escherichia coli* cells overexpressing an HslV/U protein and is stabilized by about 2-fold in an hslV/U deletion mutant. Surprisingly, however, the present inventors have further found that when the hslV/U deletion mutation is combined with deletion mutations of other ATP-dependent proteases, human prourokinase is markedly stabilized in a triple deletion mutant carrying mutations in the hslV/U gene, the clpPX gene, and the lon gene, as compared with other mutants such as the double mutant carrying mutations in the clpPX gene and the lon gene, or the hslV/U deletion mutant, or the wild type strain. The present invention has been completed based on these findings.

In sum, the present invention pertains to the following:
(1) An *Escherichia coli* mutant carrying a triple deletion mutation in the hslV/U gene, the clpPX gene, and lon gene, and possessing a function to stabilize an unstable protein expressed in *Escherichia coli*;
(2) A method for preparing the *Escherichia coli* mutant as defined in item (1) above, comprising the step of introducing a deletion mutation to the hslV/U gene of an *Escherichia coli* mutant carrying a double mutation in the clpPX gene and the lon gene;
(3) A method for stably expressing an unstable protein in *Escherichia coli*, characterized in that the *Escherichia coli* mutant as defined in item (1) above is used;
(4) A method for stabilizing an unstable protein derived from *Escherichia coli*, characterized in that the *Escherichia coli* mutant as defined in item (1) above is used;
(5) A transformant obtainable by a method comprising introducing an expression vector carrying a gene encoding a foreign protein into the *Escherichia coli* mutant as defined in item (1) above; and
(6) A method for preparing a foreign protein, characterized in that the transformant as defined in item (5) above is used.

### BRIEF DESCRIPTION OF THE DRAWINGS

The present invention will become more fully understood from the detailed description given hereinbelow and the accompanying drawings which are given by way of illustration only, and thus, are not limitative of the present invention, and wherein:
Figure 1 is a schematic view showing the clpPX operon and the lon gene on *Escherichia coli* chromosome, and a schematic view showing a deletion mutation by replacing with the cat gene sequence (HincII-NheI fragment), a nucleotide sequence between the MluI site in the clpP gene and the SalI site in the lon gene, wherein the arrows indicate the direction of transcription;
Figure 2 is a schematic view showing the hslV/U operon on *Escherichia coli* chromosome, wherein a deletion mutation takes place by replacing with the tet gene sequence (AvaI-EcoRI fragment), a nucleotide sequence between the NsiI site in the hslV gene and the NruI site in the hslU gene, wherein the arrows indicate the direction of transcription;
Figure 3 is an electrophoretic photograph showing the expression of human prourokinase visualized by Western blotting;
Figure 4 is a graph showing the results of the pulse-chase experiment for evaluating the stabilization of human prourokinase using KY2266 strain;
Figure 5 is a graph showing the Western blotting analysis for evaluating the stabilization of Cryj2 using KY2266 strain; and
Figure 6 is a graph showing the Western blotting analysis for evaluating the stabilization of σ³² using KY2893 strain.

### DETAILED DESCRIPTION OF THE INVENTION

According to the present invention, there can be provided an *Escherichia coli* mutant carrying a triple deletion mutation in the hslV/U gene, the clpPX gene, and lon gene, and possessing a function to stabilize an unstable protein expressed in *Escherichia coli*.

The term "*Escherichia coli* mutant" used herein refers to an *Escherichia coli* mutant that possesses a function to stabilize an unstable protein expressed in *Escherichia coli*. Examples of such mutants include, for instance, an *Escherichia coli* mutant carrying a triple deletion mutation in the hslV/U gene, the clpPX gene, and the lon gene, and possessing the stabilizing function described above.

The term "unstable protein" used herein refers to a protein that undergoes rapid degradation even when expressed in *Escherichia coli*, i.e., a protein having an extremely short half-life. The unstable proteins include, for instance, proteins derived from *Escherichia coli*, such as heat shock transcription factor σ³², and foreign proteins difficult to be expressed at high levels in *Escherichia coli*, such as human prourokinase, *Cryptomeria japonica* pollen antigen Cryj2, various cytokines and growth factors.

The term "stabilize or stabilizing" used herein refers to significantly extend, or significant extension of, a half-life of the protein described above. The half-life can be determined by, for instance, a pulse-chase experiment using a radiolabeled amino acid which shall be described more specifically in a subsequent section.

The triple deletion mutation described above means that by entirely or partially deleting the hslV/U gene, the clpPX gene and the lon gene, the proteases encoded thereby are made undetectable in *Escherichia coli* when visualized by, for instance, Western blotting.

Introduction of the triple deletion mutation described above on *Escherichia coli* chromosome can be confirmed by PCR.

*Escherichia coli* strains for introducing the triple deletion mutation described above are not particularly limited, and examples thereof include, for instance, K12 strain and B strain. Here, preference is given to K12 strain from the viewpoint of biological containment.

In the present invention, from the viewpoint of further stabilization of an unstable protein, particular preference is given to KY2266 strain (FERM BP-6239), obtained by a method comprising introducing a triple deletion mutation to the hslV/U gene, the clpPX gene and the lon gene of MC4100 strain [F-araDΔ(argF-lac)U169 rpsL relA flbB deoC ptsF rbsR], derived from K12 strain; and KY2893 strain (FERM BP-6243), obtained by a method comprising introducing a triple deletion mutation to the hslV/U gene, the clpPX gene and the lon gene of W3110 strain [thyA36 deoC2 IN(rrnD-rrnE)1 rph1], derived from K12 strain, and selecting a strain that can grow at a temperature of 30° to 42°C.

The method for preparing the *Escherichia coli* mutant of the present invention will be explained in detail below taking the KY2266 strain and KY2893 strain mentioned above as preferred embodiments. Unless specified otherwise, the following method can be carried out according to a method disclosed in, for instance, "Sambrook, J. et al., *Molecular Cloning: A Laboratory Manual, 2nd ed.*, Cold Spring Harbor Laboratory Press, New York, 1989, of which the disclosure is incorporated herein by reference.

First, a method for preparing KY2266 strain will be explained. KY2263 strain can be obtained by a method comprising introducing a double deletion mutation to the clpPX gene and the lon gene of the MC4100 strain mentioned above. Next, KY2266 strain carrying a triple deletion mutation in the hslV/U gene, the clpPX gene, and the lon gene can be obtained by further introducing a deletion mutation to the hslV/U gene of the resulting KY2263 strain.

### (1) Method for Preparing KY2263 strain

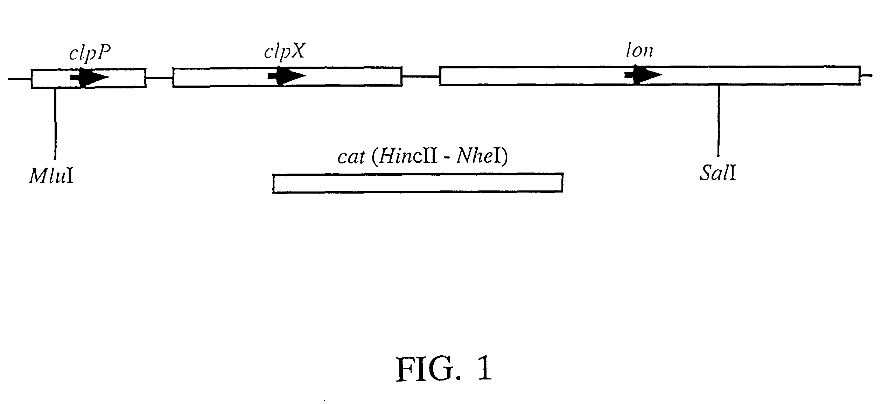

From Kohara's *Escherichia coli* ordered clone #148, the lon gene and the clpP gene, including the neighboring regions thereof, each encoding Lon(La) protease and the catalytic subunit ClpP of Clp protease, respectively, are cloned into a multicopy plasmid. The lon gene and the clpP gene are mutually located closely on the *Escherichia coli* chromosome, between which the clpX gene encoding ClpX, the regulatory subunit of the ClpXP (one of the Clp proteases), is located to constitute an operon together with the clpP gene (Figure 1). The 3.7 kbp fragment between the MluI site in the clpP gene and the SalI site in the lon gene is replaced with the chloramphenicol acetyl transferase (cat) gene (HincII-NheI fragment, 1.6 kbp) derived from pACYC184 (ATCC 37033) [Chang, A.C. and Cohen, S.N., *J. Bacteriol*. **134**, 1141-1156 (1978)] to introduce a deletion mutation (Figure 1). The obtained plasmid is named pKV1196, and the deletion mutation is named Δ(clpPX-lon)1196::cat.

FS1576 strain [Stahl, F.W. et al., *Genetics* **113**, 215-227 (1986)], a recD mutant, is transformed by using DNA linearized by cleaving the pKV1196 above with HindIII. Chromosomal DNA is prepared from a transformant acquiring chloramphenicol resistance. It is confirmed that the deletion mutation described above is introduced on the chromosome by PCR.

The Δ(clpPX-lon)1196::cat is introduced into a wild type strain MC4100 by using P1 phage [Yarmolinsky, M.B. and Sternberg, N., *The Bacteriophages* (ed. Calendar, R.), Plenum Press, New York, **1**, 291-438 (1988)]. The obtained mutant is named and identified as E. coli KY2263 strain and deposited with an accession number FERM BP-6238 with National Institute of Bioscience and Human-Technology, Agency of Industrial Science and Technology, having an address at 1-3, Higashi 1-chome, Tsukuba-shi, Ibaraki-ken 305-8566, Japan (date of original deposit: February 18, 1997; date of deposit issued pursuant to Rule 7.1 of the Budapest Treaty under the International Depositary Authority: January 26, 1998).

### (2) Method for Preparing KY2266 strain

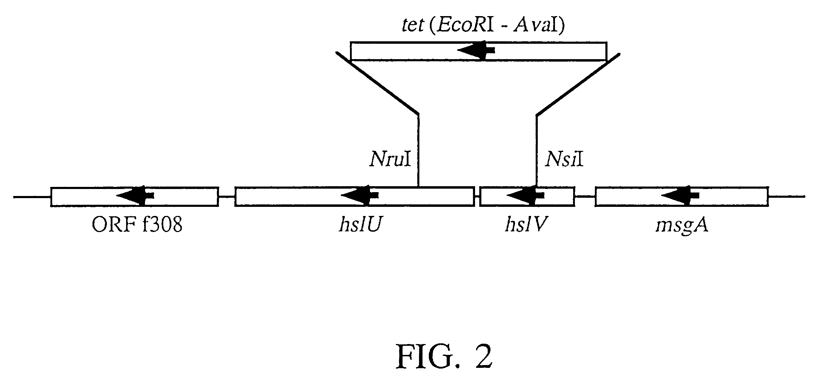

The hslV/U operon, encoding the HslV/U protease, including the neighboring regions thereof, is cloned from SG22094 strain (MC4100 strain clpP1::cat Δlon-510, Japanese Patent Laid-Open No. Hei 8-140671) into the multicopy plasmid. The 0.6 kbp fragment between the NsiI site in the hslV gene and the NruI site in the hslU gene is replaced with the tet gene (AvaI-EcoRI fragment, 1.4 kbp) derived from pBR322 (Figure 2). The obtained plasmid is named pKV1172, and the deletion mutation is named ΔhslV/U1172::tet.

FS1576 strain is transformed by using DNA linearized by cleaving the pKV1172 above with EcoRI. Chromosomal DNA is prepared from a transformant acquiring tetracycline resistance. It is confirmed that the deletion mutation described above is introduced on the chromosome by PCR.

The ΔhslV/U1172::tet is introduced in the same manner as (1) above by using P1 phage into the KY2263 strain prepared in (1) above. The obtained mutant is named and identified as E. coli KY2266 strain and deposited with an accession number FERM BP-6239 with National Institute of Bioscience and Human-Technology, Agency of Industrial Science and Technology, having an address at 1-3, Higashi 1-chome, Tsukuba-shi, Ibaraki-ken 305-8566, Japan (date of original deposit: February 18, 1997; date of deposit issued pursuant to Rule 7.1 of the Budapest Treaty under the International Depositary Authority: January 26, 1998).

KY2266 strain, the resulting *Escherichia coli* mutant prepared as above in the present invention, is advantageous in that it can be handled under ordinary procedures without substantial differences in growth rate in nutrient medium at 37°C, transformation method, storage method, and the like as compared to its parent strain MC4100 and the double mutant KY2263 strain.

The method for preparing KY2893 strain will be explained next. KY2893 strain can be prepared in the same manner as the method for KY2266 strain described above, except for using W3110 strain in place of MC4100 strain.

Specifically, first, a double deletion mutation is introduced to the clpPX gene and the lon gene of W3110 strain. The obtained mutant is named and identified as E. coli KY2783 strain and deposited with an accession number FERM BP-6244 with National Institute of Bioscience and Human-Technology, Agency of Industrial Science and Technology, having an address at 1-3, Higashi 1-chome, Tsukuba-shi, Ibaraki-ken 305-8566, Japan (date of original deposit under the Budapest Treaty: February 3, 1998).

Subsequently, KY2804 strain carrying a triple deletion mutation in the hslV/U gene, the clpPX gene, and the lon gene is obtained by introducing a deletion mutation to the hslV/U gene of KY2783 strain prepared above. Thereafter, KY2893 strain, a mutant which can grow at 30°C or a higher temperature, is separated from KY2804 strain prepared above. The obtained mutants are named and identified as E. coli KY2804 strain and E. coli KY2893 strain and deposited with an accession numbers FERM BP-6245 and FERM BP-6243, respectively, with National Institute of Bioscience and Human-Technology, Agency of Industrial Science and Technology, having an address at 1-3, Higashi 1-chome, Tsukuba-shi, Ibaraki-ken 305-8566, Japan (date of original deposit under the Budapest Treaty: February 3, 1998).

In addition, KY2893 strain, the resulting *Escherichia coli* mutant prepared as above in the present invention, is advantageous in that it can be handled under ordinary procedures without substantial differences in growth rate in nutrient medium at 37°C, transformation method, storage method, and the like as compared to its parent strain W3110 and the double mutant KY2783 strain.

Furthermore, the present invention provides a method for stably expressing an unstable protein in *Escherichia coli* using the *Escherichia coli* mutant described above. The unstable protein is preferably a foreign protein, with greater preference given to human prourokinase, *Cryptomeria japonica* pollen antigen Cryj2, and the like, because they are more significantly stably expressed by the *Escherichia coli* mutant of the present invention.

When the unstable protein is a foreign protein, the gene encoding the desired foreign protein is introduced to a vector to prepare a recombinant vector. The above vector has sequences required for expression control in the *Escherichia coli* mutant of the present invention, such as a promoter sequence, an SD sequence, an origin of replication, and a marker gene, and is exemplified by commercially available pET, pTrc99A, pKK233, and the like. A transformant expressing the desired foreign protein can be obtained by introducing the recombinant vector prepared above into the *Escherichia coli* mutant of the present invention, and selecting colonies depending upon the marker gene.

The present invention further provides the transformant described above.

The desired unstable foreign protein can be stably expressed by cultivating the transformant described above. This transformant can be cultivated by any conventional method. The medium used can be either a nutrient medium or a synthetic medium. A given cultivation temperature is chosen over the range from 20° to 42°C, and the cultivation temperature is most preferably around 37°C.

The stability of the desired foreign protein can be determined by a pulse-chase experiment comprising adding ³⁵S-methionine to a medium containing a transformant in the logarithmic growth phase to label the protein synthesized by the transformant cells for one minute, and subsequently adding non-radioactive methionine in excess. The stability of the desired foreign protein can be evaluated by its half-life.

Furthermore, the present invention provides a method for stabilizing an unstable protein derived from *Escherichia coli* using the *Escherichia coli* mutant described above. This method is hereinafter described taking as an example the heat shock transcription factor σ³² of *Escherichia coli*.

The unstable protein derived from *Escherichia coli* can be stabilized by cultivating the *Escherichia coli* mutant of the present invention in the same manner as the cultivation conditions of the transformant described above. The protein is extracted from cells of the *Escherichia coli* mutant described above by a conventional method. *The Escherichia coli* mutant having the highest expression level of σ³² can be selected by subjecting a given amount of the extracted proteins to SDS-PAGE, transferring the separated proteins onto a nitrocellulose membrane, and subsequently visualizing the protein by Western blotting using the anti-σ³² antibody (Japanese Patent Laid-Open No. Hei 8-140671).

The *Escherichia coli* mutant described above of the present invention having the highest expression level of σ³² lacks the σ³²-degrading proteases, so that not only the direct degradation of the desired proteins produced upon expression of foreign genes can be suppressed but also the desired proteins can be retained more stably, owing to the chaperone function of the large amount of heat shock proteins produced by stabilized σ³². Accordingly, the *Escherichia coli* mutant having the highest expression level of σ³² is particularly useful.

Moreover, the present invention provides a method for preparing a foreign protein using the transformant described above. The transformant described above is cultivated in the same manner as above, and induction of expression may be carried out as occasion demands. The foreign protein can be recovered and purified from the transformant by a method comprising disrupting and centrifuging the transformant, collecting the supernatant, and purifying the product by conventional methods used in protein purification, such as gel filtration and various column chromatographies [*Current Protocols in Protein Science* (ed. Coligan, J.E. et al.), John Wiley and Sons, Inc., Chapter 6]. Also, when the desired protein is present in periplasm, it can be purified by a method disclosed in Willsky et al. [*J. Bacteriol.*, **127**, 595-609 (1976)], of which the disclosure is incorporated herein by reference, and the like.

### EXAMPLES

The present invention will be explained in more detail by the following working examples, without intending to limit the scope or spirit of the present invention thereto. Unless specified otherwise, the following method was carried out according to a method disclosed in, for instance, "Sambrook, J. et al., *Molecular Cloning: A Laboratory Manual, 2nd ed*., Cold Spring Harbor Laboratory Press, New York, 1989.

### Example 1

### Preparation of KY2266 strain

From Kohara's *Escherichia coli* ordered clone #148, the lon gene and the clpP gene, including the neighboring regions thereof, each encoding Lon(La) protease and the catalytic subunit ClpP of Clp protease, respectively, were cloned into the multicopy plasmid pBR322. The lon gene and the clpP gene were mutually located closely on the *Escherichia coli* chromosome, between which the clpX gene encoding ClpX, the regulatory subunit of the ClpXP (one of the Clp proteases), was located to constitute an operon together with the clpP gene (Figure 1). The 3.7 kbp fragment between the MluI site in the clpP gene and the SalI site in the lon gene was replaced with the cat gene (HincII-NheI fragment, 1.6 kbp) derived from pACYC184 (ATCC 37033) [Chang, A.C. and Cohen, S.N., *J. Bacteriol*. **134**, 1141-1156 (1978)] to induce a deletion mutation (Figure 1). The obtained plasmid was named pKV1196, and the deletion mutation was named Δ(clpPX-lon)1196::cat.

FS1576 strain [Stahl, F.W. et al., *Genetics* **113**, 215-227 (1986)], a recD mutant, was transformed by using DNA linearized by cleaving the pKV1196 mentioned above with HindIII. Chromosomal DNA was prepared from a transformant that acquired chloramphenicol resistance. It was confirmed that the deletion mutation described above was introduced on the chromosome by PCR.

The Δ(clpPX-lon)1196::cat was introduced into a wild type strain MC4100 by using P1 phage [Yarmolinsky, M.B. and Sternberg, N., *The Bacteriophages* (ed. Calendar, R.), Plenum Press, New York, **1**, 291-438 (1988)]. The obtained mutant was named KY2263 strain (FERM BP-6238).

The hslV/U operon, encoding the HslV/U protease, including the neighboring regions thereof, was cloned from SG22094 strain (MC4100 strain clpP1::cat Δlon-510, Japanese Patent Laid-Open No. Hei 8-140671) into the multicopy plasmid described above. The 0.6 kbp fragment between the NsiI site in the hslV gene and the NruI site in the hslU gene was replaced with the tet gene (AvaI-EcoRI fragment, 1.4 kbp) derived from pBR322 (Figure 2). The obtained plasmid was named pKV1172, and the deletion mutation was named ΔhslV/U1172::tet.

FS1576 strain was transformed by using DNA linearized by cleaving the pKV1172 mentioned above with EcoRI. Chromosomal DNA was prepared from a transformant that acquired tetracycline resistance. It was confirmed that the deletion mutation described above was introduced on the chromosome by PCR.

The ΔhslV/U1172::tet was introduced by using P1 phage into KY2263 strain prepared above. The obtained mutant was named KY2266 strain (FERM BP-6239).

### Example 2

### Preparation of KY2893 strain

First, the Δ(clpPX-lon)1196::cat was introduced in the same manner as in Example 1 by using P1 phage into a wild-type strain W3110. The obtained mutant was named KY2783 strain (FERM BP-6244).

Secondly, ΔhslV/U1172::tet was introduced in the same manner as in Example 1 by using P1 phage into KY2783 strain prepared above. The obtained mutant was named KY2804 strain (FEBM BP-6245).

KY2804 strain prepared above was cultivated to grow in L-liquid medium at 42°C, and the cultivated medium was then diluted to a given concentration and spread on L-agar plates. Thereafter, the L-agar plates were incubated at 37°C or 30°C for one day. As a result, it was found that the number of colonies was lowered to one-tenth or one-ten thousandth, respectively, as compared to the colonies obtained by the cultivation at 42°C. Therefore, it is shown that KY2804 strain is cold sensitive.

Therefore, KY2804 strain prepared above was cultivated overnight at 42°C in L-liquid medium, and 2 µl of the cultivated medium was then spread on an L-agar plate and cultivated for one day at 30°C. Independent colonies without overlapping are selected from the growing colonies and streaked on a separate L-agar plate. Thereafter, the streaked colonies were cultivated again at 30°C. Similarly grown colonies were streaked on an L-agar plate and cultivated at 42°C, and a single colony was isolated, to give KY2893 strain (FERM BP-6243) which was able to grow at a temperature of 30° to 42°C.

### Example 3

### Method for stably expressing human prourokinase in KY2266 strain

It has been known that pUK-02pm0 is a multiple copy plasmid carrying the lacIq gene and the human prourokinase gene downstream of the tac promoter, and that *Escherichia coli* transformed with the above plasmid synthesizes prourokinase with dependency on the IPTG concentration in the medium [Kanemori, M. et al., *J. Bacteriol*. **176**, 5648-5653 (1994)]. Transformants were obtained by the steps of transforming MC4100 strain (wild-type strain), KY2263 strain, and KY2266 strain with the above plasmid; selecting ampicillin-resistant colonies; and confirming the expression of human prourokinase in the selected colonies by Western blotting. Each of the obtained transformants was allowed to grow logarithmically in synthetic medium (medium-E) (containing 0.2 g of MgSO₄·7H₂O, 2 g of citric acid·H₂O, 10 g of K₂HPO₄, and 3.5 g of NaNH₄HPO₄·4H₂O, per liter) at 30°C. Thereafter, IPTG was added to the synthetic medium so as to give a final concentration of 10 µM, to induce the synthesis of human prourokinase. At one hour after adding IPTG, samples were taken, and the amounts of human prourokinase were examined by Western blotting using an anti-human prourokinase antibody.

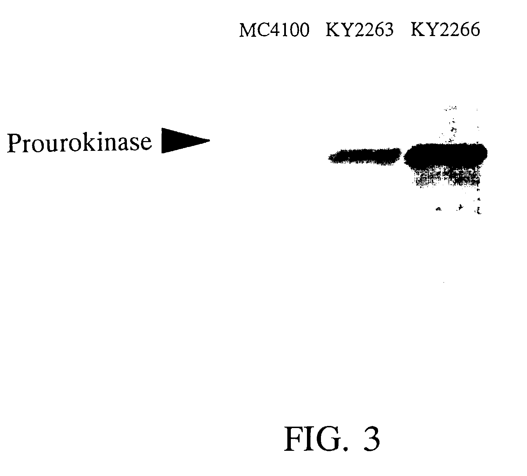

As a result, significantly increased amounts of prourokinase were observed in the KY2263 strain transformant and the KY2266 strain transformant as compared to the MC4100 strain transformant (Figure 3). Because the expression rate did not substantially change, the differences in these amounts of prourokinase are considered to reflect differences in stability. Even under the cultivation conditions for growing in nutrient medium (L-medium) (containing 10 g of Bactotrypton™ (manufactured by Difco Laboratories), 5 g of yeast extract, and 5 g of NaCl, per liter; pH 7.4) at 37°C, results similar to those described above were obtained with respect to the amounts of human prourokinase in the transformants described above.

A pulse-chase experiment was conducted by the steps of adding IPTG to medium containing transformants cultivated in the same manner as above to the logarithmic growth phase; labelling the protein synthesized in their cells for one minute with ³⁵S-methionine (100 µCi/ml); subsequently adding excess amounts of non-radioactive methionine (to give a final concentration 200 µg/ml); taking samples from each transformant at 10 minutes, 20 minutes, 30 minutes and 40 minutes, wherein one minute after adding non-radioactive methionine was set as time 0.

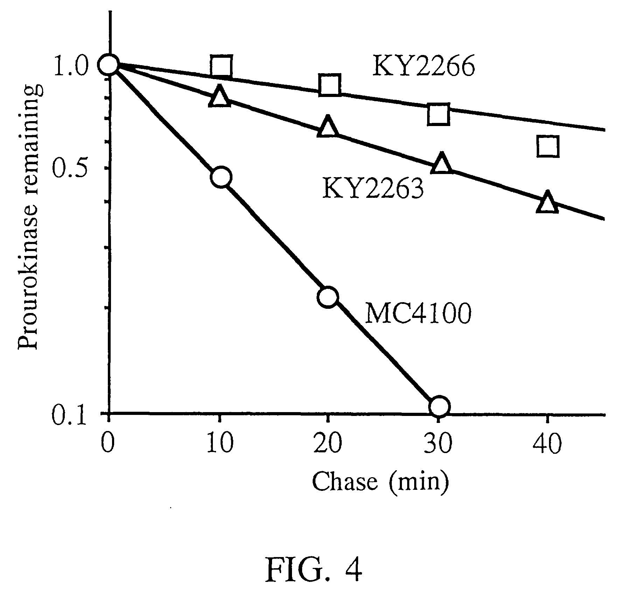

The amount of human prourokinase in each of the transformants described above was quantitatively measured by immunoprecipitation to determine the half-life of human prourokinase. As a result, the half-life was found to be 10 minutes for MC4100 strain and 30 minutes for KY2263 strain, while the half-life was 40 minutes or more for KY2266 strain, showing a significantly extended half-life in KY2266 strain (Figure 4).

### Example 4

### Method for stably expressing Cryptomeria japonica pollen antigen Cryj2 in KY2266 strain

Each of the transformants was obtained in the same manner as in Example 3 by using a Cryj2 expression vector pKCJ2I. Each of the obtained transformants was cultured in nutrient medium at 37°C. During the logarithmic growth phase, IPTG was added to the medium so as to give a final concentration of 1 mM to induce synthesis of Cryj2. After one hour, spectinomycin was added so as to give a final concentration of 500 µg/ml to stop the protein synthesis (time 0). After 5 minutes, 10 minutes, 20 minutes and 40 minutes, samples were taken from each transformant.

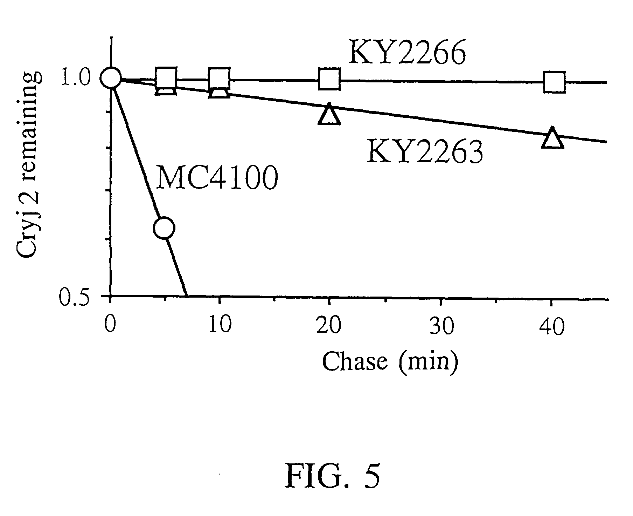

The Cryj2 in each of the transformants described above was quantitatively measured by Western blotting. As a result, MC4100 strain had a half-life of 7 minutes, while the remaining amounts of Cryj2 in KY2263 strain and KY2266 strain even after 40 minutes substantially maintained their initial levels (about 0.9 and about 1.0, respectively), thereby showing that the Cryj2 was extremely well stabilized (Figure 5).

### Example 5

### Stabilization of σ³² in KY2266 strain

A pulse-chase experiment was conducted by the steps of adding ³⁵S-methionine (100 µCi/ml) to the culture of KY2266 strain grown in synthetic medium (medium E) to the logarithmic growth phase at 30°C; labelling the protein synthesized in KY2266 strain for one minute; subsequently adding excess amounts of non-radioactive methionine (final concentration 200 µg/ml); and taking samples from KY2266 cells at 5 minutes, 10 minutes, 15 minutes and 20 minutes, wherein one minute after adding non-radioactive methionine was set as time 0. The amount of σ³² in each KY2266 cell was quantitatively measured by immunoprecipitation using an antibody against σ³² to determine the half-life of σ³². As a result, the half-life was found to be about 1.5 minutes for MC4100 strain and about 8 minutes for KY2263 strain, while the half-life was about 40 minutes for KY2266 strain, showing a significantly extended half-life in KY2266 strain.

### Example 6

### Stabilization of σ³² in KY2893 strain

Chloramphenicol was added to each culture of W3110 strain, KY2783 strain, and KY2893 strain grown in nutrient medium to the logarithmic growth phase at 37°C, so as to give a final concentration of 100 µg/ml at which point the synthesis of protein was stopped (defined as time 0). After 0 minutes, 0.5 minutes, 1.0 minutes, and 1.5 minutes (W3110 strain), or after 0 minutes, 5 minutes, 10 minutes, and 15 minutes (KY2783 strain or KY2893 strain), cell samples were taken.

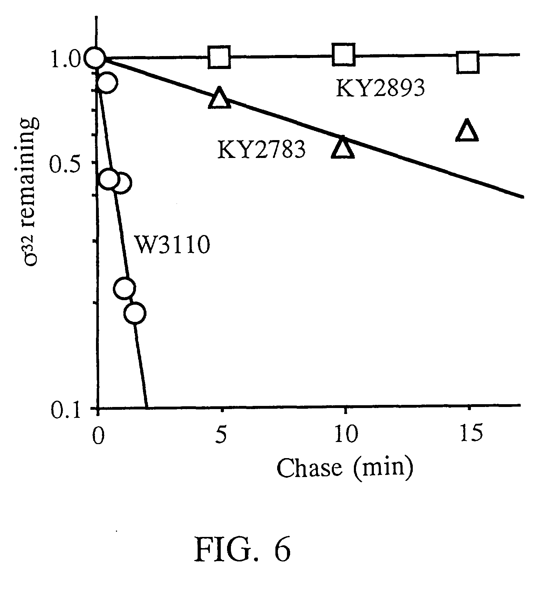

The amount of σ³² in each cell was quantitatively measured by Western blotting. As a result, KY2893 strain substantially maintained its initial level even after fifteen minutes, in contrast to the half-life of W3110 strain of about one minute, and that of KY2783 strain of about ten minutes. Therefore, it is clear from the above that the σ³² is extremely well stabilized in KY2893 strain (Figure 6).

According to the present invention, there can be provided an *Escherichia coli* mutant possessing a function to stabilize an unstable protein expressed in *Escherichia coli*, a method for preparing the *Escherichia coli* mutant, a method for stably expressing an unstable protein in *Escherichia coli* by using the *Escherichia coli* mutant, a method for stabilizing an unstable protein derived from *Escherichia coli* by using the *Escherichia coli* mutant, a transformant obtainable by a method comprising introducing an expression vector carrying a gene encoding a foreign protein into the *Escherichia coli* mutant, and a method for preparing a foreign protein using the transformant. In the present invention, an efficient production from the viewpoint of genetic engineering of useful foreign protein can be achieved.

The present invention being thus described, it will be obvious that the same may be varied in many ways. Such variations are not to be regarded as a departure from the spirit and scope of the invention, and all such modifications as would be obvious to one skilled in the art are intended to be included within the scope of the following claims.

## Claims

1. An *Escherichia coli* mutant carrying a triple deletion mutation in the hslV/U gene, the clpPX gene, and lon gene, and possessing a function to stabilize an unstable protein expressed in *Escherichia coli*.

2. The *Escherichia coli* mutant according to claim 1, wherein the *Escherichia coli* mutant is derived from K12 strain.

3. The *Escherichia coli* mutant according to claim 1, wherein the *Escherichia coli* mutant is derived from KY2266 strain (FERM BP-6239) or KY2893 strain (FERM BP-6243).

4. A method for preparing the *Escherichia coli* mutant as defined in any one of claims 1 to 3, comprising the step of introducing a deletion mutation to the hslV/U gene of an *Escherichia coli* mutant carrying a double mutation in the clpPX gene and the lon gene.

5. The method according to claim 4, wherein the *Escherichia coli* mutant carrying a double mutation in the clpPX gene and the lon gene is KY2263 strain (FERM BP-6238) or KY2783 strain (FERM BP-6244).

6. A method for stably expressing an unstable protein in *Escherichia coli*, characterized in that the *Escherichia coli* mutant as defined in any one of claims 1 to 3 is used.

7. The method according to claim 6, wherein the unstable protein is a foreign protein.

8. The method according to claim 7, wherein the foreign protein is human prourokinase.

9. The method according to claim 7, wherein the foreign protein is *Cryptomeria japonica* pollen antigen Cryj2.

10. A method for stabilizing an unstable protein derived from *Escherichia coli*, characterized in that the *Escherichia coli* mutant as defined in any one of claims 1 to 3 is used.

11. The method according to claim 10, wherein the unstable protein derived from *Escherichia coli* is a heat shock transcription factor σ³².

12. A transformant obtainable by a method comprising introducing an expression vector carrying a gene encoding a foreign protein into the *Escherichia coli* mutant as defined in any one of claims 1 to 3.

13. A method for preparing a foreign protein, characterized in that the transformant as defined in claim 12 is used.
